# EUROPEAN PATENT SPECIFICATION

(11) **EP 4 532 644 B1**
(45) Date of publication and mention of the grant of the patent: **24.12.2025**
(21) Application number: 22731206.3
(22) Date of filing: 31.05.2022
(51) Int. Cl.: C11B 9/00

(54) **ETHYL 2-METHYLBENZOATE AS A FRAGRANCE**
ETHYL-2-METHYLBENZOAT ALS EIN DUFTSTOFF
2-MÉTHYLBENZOATE D'ÉTHYLE EN TANT QUE PARFUM

(43) Date of publication of application: 09.04.2025
(73) Proprietor: Symrise AG, 37603 Holzminden Niedersachsen (DE)
(72) Inventor: HÖLSCHER, Bernd, 31785 Halle (DE); MANSFELD, Marc, 37647 Polle (DE); KLEINE-BENNE, Lara-Joy, 37603 Holzminden (DE); BELMAS, Theo, 37603 Holzminden (DE)
(74) Representative: Fabry, Bernd
(86) International application number: PCT/EP2022/064818
(87) International publication number: WO 2023/232237

(56) References cited:
- US-B2- 7 304 028
- ANONYMOUS: "Ethyl 2-methyl benzoate", 31 December 2018 (2018-12-31), XP093012793, Retrieved from the Internet <URL:https://web.archive.org/web/20191029005023/http://www.thegoodscentscompany.com/data/rw1243901.html> [retrieved on 20230110]

## Description

### FIELD OF THE INVENTION

The present invention relates to the use of ethyl 2-methylbenzoate as a fragrance. Furthermore, the present invention relates to fragrance compositions, their use and a method for imparting, modifying and/or enhancing certain fragrance notes.

### BACKGROUND OF THE INVENTION

On the part of the perfume industry, new fragrance creations are permanently needed. However, the creation of a new fragrance or fragrance compositions involves a number of challenges. For example, it is necessary to select a specific composition of a few fragrances from an almost unlimited number of possible structures known from the prior art, in order to satisfy a specific need of the market and provide a product that matches the specific profile required by the customer. Suitable fragrances show a very beneficial performance, but in combination with other fragrances may develop unpleasant and thus detrimental odor aspects, making it difficult or even impossible to use them for the specific purpose.

A second important problem concerns the need to provide fragrance compositions that are not only consistent with a particular odor profile, but also possess so-called secondary beneficial properties. Indeed, many fragrance compositions known from the market have significant drawbacks in use, such as poor solubility and stability to storage, but also failures in subjective issues such as richness, charisma, and the like. In addition, many known fragrance compositions require high dosages to achieve the desired odor result. Another requirement for fragrances today is high biodegradability as well as dermatological and toxicological safety. Consequently, there is a particularly high demand for providing fragrances that have a large effect on other fragrances even at small dosages and change rather unpleasant odor impressions into positive ones and/or enhance pleasant odor impressions.

Thus, the underlying problem of the present invention was to provide a new fragrance with the ability to enhance positive and beneficial fragrance aspects of other fragrances and / or (at the same time) to reduce, inhibit and / or mask undesirable unpleasant fragrance aspects. In particular, the new fragrance should also be characterized by improving the stability, solubility and overall performance of other fragrances, as well as reducing the required dosage. Finally, the fragrance compositions themselves should have excellent biodegradability and be harmless to humans and the environment.

### DESCRIPTION OF THE INVENTION

This is solved by the use of ethyl 2-methylbenzoate as a fragrance.

Ethyl 2-methylbenzoate to be used according to the invention may be present in any stereoisomeric form or may be present as any mixture of stereoisomers.

What has been said herein for ethyl 2-methylbenzoate, in particular the advantages described herein, also apply to a mixture of stereoisomers of ethyl 2-methylbenzoate to be used or to be employed according to the invention.

Ethyl 2-methylbenzoate possesses olfactory properties which are quite unique and which clearly differ from and also surpass those of known odoriferous substances. The suitability of ethyl 2-methylbenzoate as a fragrance was previously unknown. It is therefore particularly surprising that in the already well-studied field a fragrance with valuable, interesting and complex olfactory properties could be found.

Ethyl 2-methylbenzoate has the following structural formula 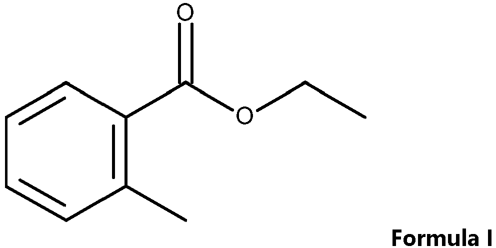 and, preferably, the following characteristic: Cas Nr.: 87-24-1

As mentioned above, its suitability as a fragrance is not known. Likewise, no precise odor description is available, however, according to the inventors, ethyl 2-methylbenzoate smells harsh, medicinal, fresh after methyl salicylate.

Consequently, the inventors have made the surprising discovery that ethyl 2-methylbenzoate is suitable as a fragrance and, in small dosages, produces special effects in combination with other fragrances, in particular fragrances having an aromatic and spicy note.

In a preferred embodiment according to the invention, ethyl 2-methylbenzoate is used in a fragrance composition to obtain at least one of the following fragrance notes: strong, clean, floral, natural, volume, fruity, fresh, spicy, durable, complex, fine, green, less sweaty, less animalic, less mushroomy and/or less terpenic. Further preferred is the use of ethyl 2-methylbenzoate for imparting, modifying and/or enhancing an aromatic and/or spicy fragrance note. Preferably, ethyl 2-methylbenzoate is used for enhancing an aromatic and/or spicy fragrance note. This is, however particularly surprising in light of the above odor description, since ethyl 2-methylbenzoate does not impart any significant odor of its own and even enhances the warm and/or spicy odor note. In a preferred embodiment according to the invention, ethyl 2-methylbenzoate strengthens aromatic and/or spicy notes of other fragrances.

The fact that ethyl 2-methylbenzoate to be used according to the invention may impart a very complex and varied overall sensory impression, which can otherwise usually only be achieved by mixtures of several components (such as essential oils or spice mixtures), is particularly surprising.

Beyond the primary, namely olfactory, properties, ethyl 2-methylbenzoate additionally possesses positive secondary properties, in particular a high substantivity compared to fragrances with similar olfactory properties, as well as a high stability in certain media and preparations, a high extensibility, and is also biodegradable.

In connection with the preferred use for imparting, modifying and/or enhancing a fragrance note, it is also recognized that ethyl 2-methylbenzoate can excellently function as a so-called booster (amplifier; enhancer). Preferably, ethyl 2-methylbenzoate is used as a booster for warm, spicy and/or aromatic notes of other fragrances. In other words: The use of ethyl 2-methylbenzoate together with other fragrances enhances their warm, spicy and/or aromatic odor notes.

Moreover, ethyl 2-methylbenzoate to be used according to the invention can enhance the intensity of a fragrance mixture (fragrance composition) and round off the overall odor of the mixture. The compound described herein can therefore be used to impart more strongness, cleanness, floralness, naturalness, volume, fruitiness, freshness, spiciness, warmness, durability, complexness, fineness, greenness, less sweatiness, less animalness, less fungi and/or less terpenic to a fragrance composition, in particular the compound can be used to impart more aroma, spiciness and/or warmness to a fragrance composition.

Furthermore, ethyl 2-methylbenzoate is suitable as an agent for increasing the substantivity and/or retention of a fragrance composition.

Ethyl 2-methylbenzoate can be used in a variety of products; it can be used as a single fragrance, but it can be combined particularly advantageously with other fragrances in different proportions to form fragrance mixtures, and novel and original perfume compositions can also be created. Accordingly, one aspect of the invention also relates to a fragrance mixture and possibly further constituents (solvents or the like), which contains ethyl 2-methylbenzoate.

The fragrance composition according to the invention comprises or consists of ethyl 2-methylbenzoate and at least one further fragrance. Preferably, the fragrance composition according to the invention comprises or consists of ethyl 2-methylbenzoate and two or more further fragrances. More preferably, the fragrance composition according to the invention comprises or consists of ethyl 2-methylbenzoate and three or more further fragrances. Most preferably, the fragrance composition according to the invention comprises or consists of ethyl 2-methylbenzoate and four or more further fragrances.

Ethyl 2-methylbenzoate according to the invention is usually used in a sensory effective amount, i.e. in a total amount in which it exerts a sensory effect.

Preferably, the weight ratio of ethyl 2-methylbenzoate to the total amount of further fragrances is in the range from 1:10 to 1:100, preferably from 1:25 to 1:50.

In this context, it is preferred if the total amount of ethyl 2-methylbenzoate is in the range from 0.0001 to 99.9% by weight, preferably from 0.001 to 99.5% by weight, particularly preferably from 0.01 to 99% by weight, from 0.01 to 90% by weight, from 0.01 to 50% by weight, from 0.01 to 20% by weight and particularly preferably from 0.01 to 1.5% by weight, in particular from 0.01 to 1.0% by weight, in each case based on the total weight of the composition.

In a preferred embodiment according to the invention, ethyl 2-methylbenzoate is contained in the fragrance composition in a sensory effective amount sufficient to modify an existing fragrance to make it stronger, cleaner, more floral, more natural, more volume, fruitier, fresher, more durable, more complex, finer, greener, less sweaty, less animalic, less mushroomy and/or less terpenic and/or to enhance an aromatic and/or spicy fragrance note. Thus, in a preferred embodiment according to the invention, ethyl 2-methylbenzoate is preferably combined with at least one aromatic and/or spicy fragrances within a fragrance composition.

Examples of fragrances which may advantageously be combined with ethyl 2-methylbenzoate in the context of the present invention can be found, for example, in S. Arctander, Perfume and Flavor Materials, Vol. I and II, Montclair, N. J. 1969, self-published, or K. Bauer ei a/., Common Fragrance and Flavor Materials, 4th Edition, Wiley-VCH, Weinheim 2001.

Specifically mentioned are: Extracts from natural raw materials such as essential oils, concretes, absolutes, resins, resinoids, balsams, tinctures such as.

Ambergris tincture; Amyris oil; Angelica seed oil; Angelica root oil; Anise oil; Valerian oil; Basil oil; tree moss absolute; bay oil; mugwort oil; benzoeresin; bergamot oil; Beeswax absolue; Birch tar oil; Bitter almond oil; Savory oil; Bucco leaf oil; Cabreuva oil; Cade oil; Calmus oil; Camphor oil; Cananga oil; Cardamom oil; Cascarilla oil; Cassia oil; Cassie-absolue; Castoreum-absolue; Cedar leaf oil; Cedarwood oil; Cistus oil; Citronella oil; Citron oil; Copaiva balsam; Copaiva balsam oil; Coriander oil; Costus root oil; Cumin oil; Cypress oil; Davana oil; Dill herb oil; Dill seed oil; Eau de brouts-Absolue; Oak moss absolute; Elemi oil; Tarragon oil; Eucalyptus citriodora oil; Eucalyptus oil; Fennel oil; Spruce needle oil; Galbanum oil; Galbanum resin; Geranium oil; Grapefruit oil; Guaiac wood oil; Gurjun balsam; Gurjun balsam oil; Helichrysum absolute; Helichrysum oil; Ginger oil; Iris root absolute; Iris root oil; Jasmine absolute; Calamus oil; Chamomile oil blue; Chamomile oil roman; Carrot seed oil; Cascarilla oil; Pine needle oil; Spearmint oil; Caraway oil; labdanum oil; labdanum absolute; labdanum resin; lavandin absolute; Lavandin oil; Lavender absolute; Lavender oil; Lemongrass oil; Lovage oil; Lime oil distilled; Lime oil pressed; Linaloe oil; Litsea cubeba oil; Bay leaf oil; Mace oil; Marjoram oil; Mandarin oil; Massoir bark oil; Mimosa absolute; Musk grain oil; Musk tincture; Muscat sage oil; Nutmeg oil; Myrrh absolute; Myrrh oil; Myrtle oil; Clove leaf oil; Clove flower oil; Neroli oil; Olibanum absolute; Olibanum oil; Opopanax oil; orange blossom absolute; orange oil; origanum oil; palmarosa oil; patchouli oil; Perilla oil; Perubalsam oil; Parsley leaf oil; Parsley seed oil; Petitgrain oil; Peppermint oil; Pepper oil; Allspice oil; Pine oil; Poley oil; Rose absolute; Rosewood oil; Rose oil; Rosemary oil; Sage oil Dalmatian; Sage oil Spanish; Sandalwood oil; Celery seed oil; Spicy lavender oil; Star anise oil; Styrax oil; Tagetes oil; Fir needle oil; Tea tree oil; Turpentine oil; Thyme oil; Tolu balsam; Tonka absolue; tuberose-absolue; vanilla extract; violet leaf-absolue; verbena oil; vetiver oil; juniper berry oil; wine yeast oil; wormwood oil; wintergreen oil; ylang oil; hyssop oil; civet-absolue; cinnamon leaf oil; cinnamon bark oil and fractions thereof, or Ingredients isolated therefrom;

Individual odorants from the group of hydrocarbons, such as 3-carene; α-pinene; β-pinene; α-terpinene; γ-terpinene; p-cymene; bisabolene; camphene; caryophyllene; cedrene; farnesene; limonene; longifolene; myrcene; ocimene; valencene; (E,Z)-1 ,3,5-undecatriene; styrene; diphenylmethane;
of the aliphatic alcohols such as Hexanol; octanol; 3-octanol; 2,6-dimethylheptanol; 2-methyl-2-heptanol; 2-methyl-2-octanol; (E)-2-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
of aliphatic aldehydes and their acetals, such as Hexanal; heptanal; octanal; nonanal; decanal; undecanal; dodecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6, 10-trimethyl-9-undecenal; 2,6, 10-trimethyl-5,9-undecadienal; heptanaldiethyl acetal; 1 , 1-dimethoxy-2,2,5-trimethyl-4-hexene; citronellyloxyacetaldehyde; 1 -( 1 -methoxy-propoxy)-(E/Z)-3-hexene;
of aliphatic ketones and their oximes such as 2-heptanone; 2-octanone; 3-octanone; 2-nonanone; 5-methyl-3-heptanone; 5-methyl-3-heptanone oxime; 2,4,4,7-tetramethyl-6-octen-3-oη; 6-methyl-5-hepten-2-one;
of aliphatic sulfur-containing compounds such as 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthen-8-thiol;
of aliphatic nitriles such as 2-nonenoic acid nitrile; 2-undecenoic acid nitrile; 2-tridecenoic acid nitrile; 3, 12-tridecadienoic acid nitrile; 3,7-dimethyl-2,6-octadienoic acid nitrile; 3,7-dimethyl-6-octenoic acid nitrile;
esters of aliphatic carboxylic acids, e.g. (E)- and (Z)-3-hexenyl formate; ethyl acetoacetate; isoamyl acetate; 3,5,5-trimethylhexyl acetate; 3-methyl-2-butenyl acetate; (E)-2-hexenyl acetate; (E)- and (Z)-3-hexenyl acetate; Octyl acetate; 3-octyl acetate; 1-octene-3-yl acetate; ethyl butyrate; butyl butyrate; isoamyl butyrate; hexyl butyrate; (E)- and (Z)-3-hexenyl isobutyrate; Hexyl crotonate; ethyl isovalerate; ethyl 2-methylpentanoate; ethyl hexanoate; allyl hexanoate; ethyl heptanoate; allyl heptanoate; ethyl octanoate; Ethyl (E,Z)-2,4-decadienoate; methyl 2-octinate; methyl 2-noninate; allyl 2-isoamyloxyacetate; M ethy 1-3,7-dimethyl-2,6-octadiene-oate;4-methyl-2-pentyl crotonate;
of acyclic terpene alcohols such as. Geraniol; nerol; lavadulol; nerolidol; farnesol; tetrahydrolinalool; tetrahydrogeraniol; 2,2-Dimethyl-3-(3-methylphenyl)propan-1-ol; 2,6-dimethyl-7-octen-2-ol; 2,6-dimethyloctan-2-ol; 2- methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-Dimethyloct-6-en-1-ol; (2E)-3,7-Dimethylocta-2,6-dien-1-ol; 3,7-dimethyl-1 ,5,7-octatrien-3-ol 2,6-dimethyl-2,5J-octatrien-1-ol; and their formates, acetates, propionates, isobutyrates, butyrates, isovalerianates, pentanoates, hexanoates, crotonates, tiglinates and 3-methyl-2-butenoates;
of the acyclic terpene aldehydes and ketones such as citronellal; 7-methoxy-3,7-dimethyloctanal; 2,6,10-trimethyl-9-undecenal; geranylacetone; and the dimethyl and diethylacetals of geranial, neral,
of cyclic terpene alcohols such as. menthol; isopulegol; alpha-terpineol; terpinenol-4; menthan-8-ol; menthan-1-ol; menthan-7-ol; borneol; isoborneol; linalool oxide; nopol; cedrol; ambrinol; vetiverol; guaiaol; and their formates, acetates, propionates, isobutyrates, butyrates, isovalerianates, pentanoates, hexanoates, crotonates, tiglinates and 3-methyl-2-butenoates;
of cyclic terpene aldehydes and ketones such as Menthone; isomenthone; 8-mercaptomenthan-3-oη; carvone; camphor; fenchone; alpha-lonone; beta-lonone; alpha-n-methylionone; beta-n-methylionone; alpha-Isomethylionone; beta-Isomethylionone; alpha-irone; beta-damascenone; 1-(2,4,4-trimethyl-2-cyclohexen-1-yl)-2-buten-1-one; 1 ,3,4,6,7,8a-Hexahydro-1 , 1 ,5,5-tetramethyl-2H-2,4a-methano-naphthalen-8(5H)-on;2-Methyl-4-(2,6,6-trimethyl-1-cyclohexen-1-yl)-2-butenal; Nootkatone; dihydronootkatone; 4,6,8-megastigmatriene-3-oη; alpha-sinensal; beta-sinensal; acetylated cedarwood oil (methylcedrylketone);
of cyclic alcohols such as 4-tert-butylcyclohexanol; 3,3,5-trimethylcyclohexanol; 3-Isocamphylcyclohexanol; 2,6,9-trimethyl-Z2,Z5,E9-cyclododecatrien-1-ol; 2-Isobutyl-4-m ethyltetrahyd ro-2H-pyran-4-ol; 4-cyclohexylbutan-2-ol;
of cycloaliphatic alcohols such as. alpha,3,3-trimethylcyclohexylmethanol; 1-(4-Isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 3-methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
cyclic and cycloaliphatic ethers, e.g. Cineol; cedryl methyl ether; cyclododecyl methyl ether;1 , 1-dimethoxycyclododecane; (ethoxymethoxy)cyclo-dodecane; alpha-cedrene epoxide; 3a,6,6,9a-tetramethyldodecahydro-naphtho[2, 1-b]furan; 3a-ethyl-6,6,9a-trimethyldodecahydronaphtho[2, 1 b]furan; 1 ,5,9-tri-methyl-13-oxabicyclo[10. 1 .0]trideca-4,8-diene; rose oxide; 2-(2,4-dimethyl-3-cyclohexen-1-yl)-5-m ethyl-5-( 1 - methyl propyl)- 1 , 3-d ioxane;
of cyclic and macrocyclic ketones such as 4-tert.- butylcyclohexanone ; 2,2,5-trimethyl-5-pentylcyclopentanone ; 2-heptylcyclopentanone ; 2-pentyl-cyclopentanone ; 2-hyd roxy-3-m ethyl-2-cyclopenten- 1 -one ; 3-methyl-cis-2-penten- 1 -yl-2-cyclopenten- 1 -one ; 3-methyl-2-pentyl-2-cyclopenten-1-one ; 3-methyl-4-cyclopentadecenone ; 3-methyl-5-cyclopentadecenone ; 3-methylcyclopenta-decanone ; 4-(1-ethoxyvinyl)-3, 3,5,5-tetramethylcyclohexanone ; 4-tert. - pentylcyclo-hexanone; 5-cyclohexadecen-1-one; 6,7-dihydro-1 , 1 ,2,3,3-pentamethyl-4(5H)-indanone; 8-cyclohexadecen-1-one; 9-cycloheptadecen-1-oη; cyclopentadecanone; cyclohexadecanone;
of cycloaliphatic aldehydes such as 2-methyl-4-(2,2,6-trimethyl-cyclohexen-1-yl)-2-butenal; 4-(4-hydroxy-4-methylpentyl)-3-cyclohexenecarbaldehyde; 4-(4-methyl-3-penten-1-yl )-3-cyclohexenecarbald ehyde ;
of cycloaliphatic ketones, e.g. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2, 3,8,8-tetramethyl-1 , 2,3,4,5,6,7, 8-octahydro-2-naphthalenyl methyl ketone; methyl 2,6, 10-trimethyl-2,5,9-cyclododecatrienyl ketone; tert. - butyl-(2,4-dimethyl-3-cyclohexen-1-yl)ketone;
of ester of cyclic alcohols such as 2-tert-butylcyclohexyl acetate; 4-tert-butylcyclohexyl acetate; 2-tert-pentylcyclohexyl acetate; 4-tert-pentylcyclohexyl acetate; 3,3, 5-tri methylcyclohexyl acetate; decahyd ro-2-naphthyl acetate; 2-cyclo-pentylcyclopentyl crotonate; 3-pentyl tetrahydro-2H-pyran-4-yl acetate; decahydro-2,5,5,8a-tetramethyl-2-naphthyl acetate; 4,7-methano-3a,4,5,6,7,7a-hexa-hydro-5, resp. 6-indenyl acetate; 4,7-methano-3a,4,5,6,7,7a-hexahydro-5, or 6-indenyl propionate; 4,7-methano-3a,4,5,6,7,7a-hexahydro-5, or 6-indenyl isobutyrate; 4,7-methanooctahydro-5, or 6-indenyl acetate;
of esters of cycloaliphatic alcohols such as 1-cyclohexylethyl crotonate;
of esters of cycloaliphatic carboxylic acids, e.g. Allyl 3-cyclohexyl propionate; allyl cyclohexyloxy acetate; ice and trans-methyl dihydrojasmonate; ice and trans-methyl jasmonate; methyl 2-hexyl-3-oxocyclopentane carboxylate; ethyl 2-ethyl-6,6-dimethyl-2-cyclohexene carboxylate; ethyl 2,3,6,6-tetramethyl-2-cyclohexene carboxylate; ethyl 2-methyl-1 ,3-dioxolane-2-acetate;
of araliphatic alcohols such as Benzyl alcohol; 2-phenylethanol; 1-phenylethyl alcohol; 3-phenylpropanol; 2-phenylpropanol; 2-phenoxyethanol; 2,2-dimethyl-3-phenylpropanol; 2,2-dimethyl-3-(3-methylphenyl)propanol; 1 , 1-dimethyl-2-phenylethyl alcohol; 1 , 1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-Isopropylphenyl)ethanol;
of esters of araliphatic alcohols and aliphatic carboxylic acids, e.g. Benzyl acetate; benzyl propionate; benzyl isobutyrate; benzyl isovalerate; 2-phenylethyl acetate; 2-phenylethyl propionate; 2-phenylethyl isobutyrate; 2-phenyl ethyl isovalerate; 1-phenylethyl acetate; alpha-trichloromethyl benzyl acetate; alpha, alpha-dimethylphenyl ethyl acetate; alpha,alpha-dimethylphenyl ethyl butyrate; cinnamyl acetate; 2-phenoxyethyl isobutyrate; 4-methoxybenzyl acetate;
of araliphatic ethers such as. 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1 ,3-dioxane; 4,4a,5,9b-tetra-hydroindeno[1 ,2-d]-m-dioxin; 4,4a,5,9b-tetrahydro-2,4-dimethylindeno[1 ,2-d]-m-dioxin; of aromatic and araliphatic aldehydes such as. e.g., benzaldehyde; phenylacetaldehyde; 3-phenylpropanal; hydratropaaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-isobutylphenyl)propanal; 3-(4-tert. butylphenyl)propanal; cinnamaldehyde; alpha-butylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
of aromatic and araliphatic ketones such as acetophenone; 4-methylacetophenone; 4-methoxyacetophenone; 4-tert.- butyl-2,6-dimethylaceto-phenone; 4-phenyl-2-butanone; 4-(4-hydroxyphenyl)-2-butanone; 1-(2-naphtha-lenyl)ethanone;2-benzofuranylethanone;(3-methyl-2-benzofuranyl)ethanone; benzophenone; 1 , 1 ,2,3,6-hexamethyl-5-indanyl methyl ketone; 6-tert. butyl-1 , 1-di-methyl-4-indanyl methyl ketone; 1-[2,3-dihydro-1 , 1 ,2,6-tetramethyl-3-(1-methylethyl) -1 H-5-indenyl]ethanone; 5',6',7',8'-tetrahydro-3',5',5',6',8',8'-hexamethyl-2-aceto-naphthone;
of aromatic and araliphatic carboxylic acids and their esters, e.g. Benzoic acid; phenylacetic acid; methyl benzoate; ethyl benzoate; hexyl benzoate; benzyl benzoate; methyl phenyl acetate; ethyl phenyl acetate; geranyl phenyl acetate; phenyl ethyl phenyl acetate; methyl cinnamate; ethyl cinnamate; benzyl cinnamate; phenyl ethyl cinnamate; cinnamyl cinnamate; allyl phenoxy acetate; methyl salicylate; hexyl salicylate; cyclohexyl salicylate; cis-3-hexenyl salicylate; benzyl salicylate; phenyl ethyl salicylate; methyl 2,4-dihydroxy-3,6-dimethylbenzoate; ethyl 3-phenyl glycidate; ethyl 3-methyl-3-phenyl glycidate;
of heterocyclic compounds such as 2,5-dimethyl-4-hydroxy-2H-furan-3-one ; 2-ethyl-4-hyd roxy-5-methyl-2H-f u ran-3-oη ; 3-hyd roxy-2-m ethyl-4H-pyran-4-one ; 2-ethyl-3-hydroxy-4H-pyran-4-one;
of lactones such as. 1 ,4-octanolide; 3-methyl-1 ,4-octanolide; 1 ,4-nonanolide; 1 ,4-decanolide; 8-decen-1 ,4-olide; 1 ,4-undecanolide; 1 ,4-dodecanolide; 1 ,5-decanolide; 1 ,5-dodecanolide;4-methyl-1 ,4-decanolide; 1 , 15-pentadecanolide; 1 , 16-hexadecanolide; 9-hexadecene-1 ,16-olide; 10-oxa-1 ,16-hexadecanolide; 1 1-oxa-1 ,16-hexadecanolide; 12-oxa-1 ,16-hexadecanolide; ethylene-1 ,12-dodecanedioate; ethylene-1 ,13-tridecanedioate; 2,3-dihydrocoumarin; octahydrocoumarin.

In a further preferred embodiment of the invention, ethyl 2-methylbenzoate is preferably combined with one or more, particularly preferably with two, three, four, five or more other fragrances that have an aromatic and/or spicy odor note.

Correspondingly, the present invention also relates to a fragrance composition which comprises one, two, three, four, five or more other fragrances which convey a spicy and/or aromatic odor note.

Ethyl 2-methylbenzoateto be used according to the invention advantageously (at least partially) achieves an odor enhancement of the spicy and/or aromatic notes.

In a preferred embodiment according to the invention, the at least one further fragrance is selected from the group consisting of (1R,2S,4R)-1,7,7-Trimethyl-bicyclo[2.2.1]heptan-2-ol, (1S,2R,4S)-1,7,7-Trimethyl-bicyclo[2.2.1]heptan-2-ol, (1S,2S,4S)-1,7,7-Trimethyl-bicyclo[2.2.1]heptan-2-ol, (1R,2R,4R)-1,7,7-Trimethyl-bicyclo[2.2.1]heptan-2-ol, [2-(2-methylbutan-2-yl)cyclohexyl] acetate, Terpinen-4-ol, Isobornyl Acetate, 6,6-Dimethyl-2-methylidenebicyclo[3.1.1]heptanePin-2(10)-ene, (1S,5S)-2,6,6-Trimethylbicyclo[3.1.1]hept-2-ene ((-)-α-Pinene), 1,7,7-Trimethylbicyclo[2.2.1]heptan-2-one, Anethol, Cinnamic Aldehyde, Methyl-4(or 1)-isopropyl-1(or 4)-methylbicyclo[2.2.2]oct-5-ene-2-carboxylate, (1R,4E,9S)-4,11,11-Trimethyl-8-methylidenebicyclo[7.2.0]undec-4-ene, Isoeugenyl Methyl Ether, 4-Allyl-2-methoxyphenol, 2-butyl-4,4,6-trimethyl-1,3-dioxane, Cuminic Aldehyde, 2,3,3-Trimethylindan-1-one, Pinanol, Dihydro Myrcenol, Terpinyl acetate, 3-methyl-5-phenylpent-2-enenitrile, Geranyl acetate, benzaldehyde, ionone alpha, benzyl salicylate, phenylethyl alcohol, 3,7-Dimethyloct-6-en-1-ol, Methyldihydrojasmonat, (5E)-3-methylcyclopentadec-5-en-1-one, (3aR,5aS,9aS,9bR)-3a,6,6,9a-Tetramethyldodecahydronaphtho[2,1-b]furan, 3-Ethoxy-4-hydroxybenzaldehyd, 4-Hydroxy-3-methoxybenzaldehyd.

Preferably, ethyl 2-methylbenzoate is combined with one or more, particularly preferably with two, three, four, five or more of those preferred other fragrances.

Perfume oil compositions (=fragrance mixtures) containing 4-cyclohexyl-2-butanol are advantageously used for perfuming in liquid form, undiluted or diluted with a solvent. Suitable solvents for this are e.g. Ethanol, isopropanol, diethylene glycol monoethyl ether, glycerin, propylene glycol, 1, 2-butylene glycol, dipropylene glycol, diethyl phthalate, triethyl citrate, isopropyimyristate etc.

Furthermore, fragrance compositions according to the invention can be adsorbed on a carrier, which ensures both a fine distribution of the fragrances in the product and a controlled release during use. Such carriers can be porous inorganic materials such as light sulfate, silica gels, zeolites, plasters, clays, clay granules, aerated concrete, etc. or organic materials such as wood, cellulose-based substances, sugar, dextrins (e.g. maltodextrin) or plastics such as PVC, polyvinyl acetates or polyurethanes. The resulting combination of compositions according to the invention and carrier substance is also to be understood as a fragrance composition according to the invention.

Fragrance compositions according to the invention can also be microencapsulated, spray-dried, as inclusion complexes or as extrusion products and added in this form to a product to be perfumed, for example.

If necessary, the properties of the compositions modified in this way can be further optimized by so-called "coating" with suitable materials with a view to a more targeted release of fragrances, for which purpose waxy plastics such as polyvinyl alcohol are preferably used. The resulting products in turn represent articles according to the invention.

A further aspect of the present invention relates to the use of the fragrance composition to modify an existing fragrance within the fragrance composition to make it stronger, cleaner, more floral, more natural, more volume, fruitier, fresher, more durable, more complex, finer, greener, less sweaty, less animalic, less mushroomy and/or less terpenic and/or to enhance an aromatic and/or spicy fragrance note of the existing fragrance. Thus, in a preferred embodiment according to the invention, ethyl 2-methylbenzoate is preferably used together with at least one aromatic and/or spicy fragrance within a fragrance composition to enhance the spicy and/or aromatic notes of the at least one aromatic and/or spicy fragrance.

Preferably, the existing fragrance is selected from the fragrances listed above. More preferably, the existing fragrance is selected from the group consisting of (1R,2S,4R)-1,7,7-Trimethyl-bicyclo[2.2.1]heptan-2-ol, (1S,2R,4S)-1,7,7-Trimethyl-bicyclo[2.2.1]heptan-2-ol, (1S,2S,4S)-1,7,7-Trimethyl-bicyclo[2.2.1]heptan-2-ol, (1R,2R,4R)-1,7,7-Trimethyl-bicyclo[2.2.1]heptan-2-ol, [2-(2-methylbutan-2-yl)cyclohexyl] acetate, Terpinen-4-ol, Isobornyl Acetate, 6,6-Dimethyl-2-methylidenebicyclo[3.1.1]heptanePin-2(10)-ene, (1S,5S)-2,6,6-Trimethylbicyclo[3.1.1]hept-2-ene ((-)-α-Pinene), 1,7,7-Trimethylbicyclo[2.2.1]heptan-2-one, Anethol, Cinnamic Aldehyde, Methyl-4(or 1)-isopropyl-1(or 4)-methylbicyclo[2.2.2]oct-5-ene-2-carboxylate, (1R,4E,9S)-4,11,11-Trimethyl-8-methylidenebicyclo[7.2.0]undec-4-ene, Isoeugenyl Methyl Ether, 4-Allyl-2-methoxyphenol, 2-butyl-4,4,6-trimethyl-1,3-dioxane, Cuminic Aldehyde, 2,3,3-Trimethylindan-1-one, Pinanol, Dihydro Myrcenol, Terpinyl acetate, 3-methyl-5-phenylpent-2-enenitrile, Geranyl acetate, benzaldehyde, ionone alpha, benzyl salicylate, phenylethyl alcohol, 3,7-Dimethyloct-6-en-1-ol, Methyldihydrojasmonat, (5E)-3-methylcyclopentadec-5-en-1-one, (3aR,5aS,9aS,9bR)-3a,6,6,9a-Tetramethyldodecahydronaphtho[2,1-b]furan, 3-Ethoxy-4-hydroxybenzaldehyd, 4-Hydroxy-3-methoxybenzaldehyd.

Further preferred embodiments listed above also apply to the use of the fragrance composition.

Fragrance compositions according to the invention can advantageously be used in concentrated form, in solutions or in the modified form described above for the production of perfumed articles according to the invention, such as. B. perfume extracts, eau de perfumes, eau de toilettes, aftershave, eau de colognes, pre-shave products, splash colognes and perfumed refreshing towels and the perfuming of acidic, alkaline and neutral cleaning agents such as floor cleaners, window glass cleaners, dishwashing detergents , Bathroom and sanitary cleaners, scouring milk, solid and liquid toilet cleaners, powder and foam carpet cleaners, textile fresheners, ironing aids, liquid detergents, powder detergents, laundry pretreatment agents such as bleaches, soaking agents and stain removers, fabric softeners, laundry soaps, washing tablets, and disinfectants Air fresheners in liquid, gel-like or applied form on a solid carrier, aerosol sprays, waxes and polishes such as furniture polishes, floor waxes, shoe creams and personal care products such as solid and liquid soaps, shower gels, shampoos, shaving soaps, shaving foams s, bath oils, cosmetic emulsions of the oil-in-water, of the water-in-oil and of the water-in-oil-in-water type such as, for example, skin creams and lotions, face creams and lotions, sun protection creams and lotions, after-sun creams and lotions, hand creams and lotions, foot creams and lotions, depilatory creams and lotions, after-shave creams and lotions, tanning creams and lotions, hair care products such as hair sprays, hair gels, setting hair lotions , Hair conditioners, permanent and semi-permanent hair dyes, hair shaping agents such as cold waves and hair straighteners, hair lotions, hair creams and lotions, deodorants and antiperspirants such as underarm sprays, roll-ons, deodorant sticks, deodorant creams, products in decorative cosmetics such as eye shadow, nail varnish, Lipsticks, mascara and candles, lamp oils, incense sticks, insecticides, repellants and fuels.

Of course, fragrance compositions according to the invention can also be included in cosmetic compositions or household compositions.

Another aspect of the invention relates to a method for modifying an existing fragrance to make it stronger, cleaner, more floral, more natural, more volume, fruitier, fresher, more durable, more complex, finer, greener, less sweaty, less animalic, less mushroomy and/or less terpenic and/or to enhance a spicy and/or aromatic fragrance note, comprising or consisting of the following steps:
(a) providing ethyl 2-methylbenzoate,
(b) providing at least one further fragrance,
(c) adding the ethyl 2-methylbenzoate to the at least one further fragrance in a sensory effective amount sufficient to modify an existing fragrance to make it stronger, cleaner, more floral, more natural, more volume, fruitier, fresher, more durable, more complex, finer, greener, less sweaty, less animalic, less mushroomy and/or less terpenic and/or to enhance a spicy and/or aromatic fragrance note.

Preferably, the at least one further fragrance and the existing fragrance are the same compound(s). Thus, in a preferred embodiment according to the invention, ethyl 2-methylbenzoate is added to the at least one further fragrance in a sensory effective amount sufficient to modify the at least one further fragrance to make it stronger, cleaner, more floral, more natural, more volume, fruitier, fresher, more durable, more complex, finer, greener, less sweaty, less animalic, less mushroomy and/or less terpenic and/or to enhance a spicy and/or aromatic fragrance note.

Preferably, the weight ratio of ethyl 2-methylbenzoate to the total amount of further fragrances is in the range 1:10 to 1:100, preferably from 1:25 to 1:50.

In this context, it is preferred if the total amount of ethyl 2-methylbenzoate is in the range from 0.0001 to 99.9% by weight, preferably from 0.001 to 99.5% by weight, particularly preferably from 0.01 to 99% by weight, from 0.01 to 90% by weight, from 0.01 to 50% by weight, from 0.01 to 20% by weight and particularly preferably from 0.01 to 1.5% by weight, in particular from 0.01 to 1.0% by weight, in each case based on the total weight of the composition

Preferably, ethyl 2-methylbenzoate is combined with one or more, particularly preferably with two, three, four, five or more other fragrances that have a spicy and/or aromatic odor note.

Ethyl 2-methylbenzoate to be used according to the invention advantageously (at least partially) achieves an odor enhancement of the spicy and/or aromatic fragrance notes.

In a preferred embodiment according to the invention, the at least one further fragrance is selected from the group consisting of (1R,2S,4R)-1,7,7-Trimethyl-bicyclo[2.2.1]heptan-2-ol, (1S,2R,4S)-1,7,7-Trimethyl-bicyclo[2.2.1]heptan-2-ol, (1S,2S,4S)-1,7,7-Trimethyl-bicyclo[2.2.1]heptan-2-ol, (1R,2R,4R)-1,7,7-Trimethyl-bicyclo[2.2.1]heptan-2-ol, [2-(2-methylbutan-2-yl)cyclohexyl] acetate, Terpinen-4-ol, Isobornyl Acetate, 6,6-Dimethyl-2-methylidenebicyclo[3.1.1]heptanePin-2(10)-ene, (1S,5S)-2,6,6-Trimethylbicyclo[3.1.1]hept-2-ene ((-)-α-Pinene), 1,7,7-Trimethylbicyclo[2.2.1]heptan-2-one, Anethol, Cinnamic Aldehyde, Methyl-4(or 1)-isopropyl-1(or 4)-methylbicyclo[2.2.2]oct-5-ene-2-carboxylate, (1R,4E,9S)-4,11,11-Trimethyl-8-methylidenebicyclo[7.2.0]undec-4-ene, Isoeugenyl Methyl Ether, 4-Allyl-2-methoxyphenol, 2-butyl-4,4,6-trimethyl-1,3-dioxane, Cuminic Aldehyde, 2,3,3-Trimethylindan-1-one, Pinanol, Dihydro Myrcenol, Terpinyl acetate, 3-methyl-5-phenylpent-2-enenitrile, Geranyl acetate, benzaldehyde, ionone alpha, benzyl salicylate, phenylethyl alcohol, 3,7-Dimethyloct-6-en-1-ol, Methyldihydrojasmonat, (5E)-3-methylcyclopentadec-5-en-1-one, (3aR,5aS,9aS,9bR)-3a,6,6,9a-Tetramethyldodecahydronaphtho[2,1-b]furan, 3-Ethoxy-4-hydroxybenzaldehyd, 4-Hydroxy-3-methoxybenzaldehyd.

Preferably, ethyl 2-methylbenzoate is combined with one or more, particularly preferably with two, three, four, five or more of those preferred other fragrances.

Further preferred embodiments listed above also apply to method of the present invention.

A further aspect of the present invention relates to a perfumed product comprising ethyl 2-methylbenzoate and two or more other fragrances and optionally other ingredients, characterized in that the blend comprises ethyl 2-methylbenzoate in a quantity which
(a) modifies the olfactory properties of the two or more other fragrances to make it stronger, cleaner, more floral, more natural, more volume, fruitier, fresher, more durable, more complex, finer, greener, less sweaty, less animalic, less mushroomy and/or less terpenic and/or
(b) enhances the spicy and/or aromatic notes of the olfactory properties of the two or more other fragrances.

In a preferred embodiment the perfumed product is selected from cosmetic, hygiene and/or household articles. Further preferred embodiments listed above also apply to the perfumed product of the present invention.

Preferably, the perfumed article is selected from the group consisting of detergents and cleaning agents, hygiene or care products, preferably in the field of body and hair care, cosmetics and household, preferably from the group consisting of perfume extracts, Eau de perfumes, eau de toilets, aftershave lotions, eau de colognes, pre-shave products, splash colognes, perfumed refreshing tissues, acidic, alkaline or neutral cleaning agents, textile fresheners, ironing aids, liquid detergents, powder detergents, laundry pretreatment agents, fabric softeners, Wash tablets, disinfectants, surface disinfectants, air fresheners, aerosol sprays, waxes and polishes, personal care products, hand creams and lotions, foot creams and lotions, depilatory creams and lotions, after shave creams and lotions, tanning creams and lotions, hair care products, hair care products , Products of the decorative cosmetics, candles, lamp oils, incense sticks, insecticides, repellants and fuels. Most preferably, the perfumed product is selected from alcoholic perfume, a personal hygiene product or a cleaning or care product for use in the home.

In addition, it is preferred for the perfumed product according to the invention that ethyl 2-methylbenzoate is contained in a sensory effective amount which is sufficient for a consumer to have one or more olfactory properties selected from the group consisting of stronger, cleaner, more floral, more natural, more volume, fruitier, fresher, more durable, more complex, finer, greener, less sweaty, less animalic, less mushroomy and/or less terpenic and/or enhanced spicy and/or aromatic odor note. It is also preferred that the total amount of 4 ethyl 2-methylbenzoate, based on the total weight of the perfumed product, is in the range from 0.00001 to 10% by weight, preferably from 0.0001 to 5% by weight %, particularly preferably 0.001 to 2% by weight, more preferably 0.005 to 1% by weight.

The additives, auxiliaries and / or active substances described above are preferably not odoriferous substances. These can be, for example, preservatives, antibacterial agents, chelating agents, cleaning agents, emulsifiers, fats, etc. and in principle all substances that are used as additives, auxiliaries and / or active ingredients in cosmetics, especially in fragrance compositions, as well as in household compositions.

In the following, the invention is further characterized on the basis of the examples.

### EXAMPLES

### Example 1: Synthesis of ethyl 2-methylbenzoate

Ethyl 2-methylbenzoate according to Formula I can be manufactured according to known methods described in the art. 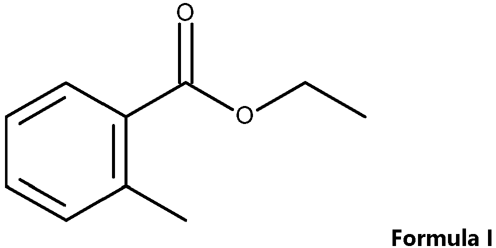 Cas Nr.: 87-24-1
Odor of ethyl 2-methylbenzoate: harsh, medicinal, fresh after methyl salicylate.

### Example 2: Odor description of preferred fragrances after addition of ethyl 2-methylbenzoate to fragrances with a spicy and/or aromatic odor note

**Table1: Odor description with addition of ethyl 2-methylbenzoate. All quantities are given in weight percentage**

| **Nr.:** | **Name** | **Amount of ethyl 2-methylbenzoate** | **Odor description** |
|---|---|---|---|
| **1** | **Borneol L/Isoborneol 65/35 10% DPG** | 0,03% | -Less sweaty |
| | | | -stronger and cleaner |
| **2** | **Coniferan** | 0,1% | -Damascone and plum note |
| | | | -stronger and less camphor |
| **3** | **Terpinenol-4 Nat.** | 0,4% | -less animalistic |
| | | | -more floral |
| | | | -less mushroomy and earthy |
| **4** | **Isobornyl Acetate** | 0,2% | -more natural (spruce needle) |
| | | | -Agrumex apple note |
| | | | -more volume and damascone note |
| **5** | **Pinene Beta Nat** | 0,2% | -stronger in the mango peel note |
| **6** | **Pinene Alpha Laevo Nat.** | 0,2% | -less terpenic |
| | | | -more natural (cypress) |
| | | | -stronger |
| **7** | **Camphor DL 10% DPG** | 0,04% | -stronger in the patchouli note |
| | | | -more volume |
| | | | -less sweaty |
| **8** | **Anethol Supra 21,5 Celsius** | 0,3% | -more natural (star anise/fennel) |
| | | | -stronger & more durable |
| **9** | **Cinnamic Aldehyde** | 0,2% | -fresher |
| | | | -more natural (cinnamon bark) |
| | | | -more radiation |
| **10** | **Poivrol^{®} (Methyl-4(or 1)-isopropyl-1(or 4)-methylbicyclo [2.2.2]oct-5-ene-2-carboxylate)** | 0,1% | -natural (black peppercorns) |
| | | | -spicier & hotter |
| | | | -more durable |
| **11** | **Caryophyllene Nat. Rect.** | 0,1% | -stronger carrot & copaiba balm |
| | | | -more complex & finer |
| **12** | **Isoeugenyl Methyl Ether** | 0,2% | -stronger in the bay note |
| **13** | **Eugenol Nat.** | 0,2% | -more natural (carnation & bay) |
| | | | -more metallic |
| | | | -powdery |
| | | | -Booster for the warm notes |
| **14** | **Herboxan** | 0,1% | -stronger |
| | | | -greener |
| | | | -strengthens the spicy, warm cumin note |
| **15** | **Cuminic Aldehyde** | 0,5% | -less greasy |
| | | | -natural (cumin) |
| **16** | **Safraleine** | 0,2% | -natural (saffron) |
| | | | -leather |
| | | | -stronger |
| | | | -Birch tar note |

### Example 3: Perfume oils with spicy and/or aromatic odor notes

| | |
|---|---|
| BUTYL BUTYRYL ACETATE | 30,000 |
| TERPINYL ACETATE | 3,000 |
| ORANGE OIL | 10,000 |
| PEPPER BLACK FRESH OIL MADA | 2,000 |
| NUTMEG OIL EXTRA | 8,000 |
| JASMAPRUNAT | 3,000 |
| ETHYL BUTYRATE | 5,000 |
| ISOAMYL BUTYRATE | 2,000 |
| ALDEHYDE C14 SO-CALLED | 7,000 |
| ETHYL MALTOL | 25,000 |
| MALTOL | 10,000 |
| METHYL CYCLOPENTENOLONE-3,2,2 | 3,000 |
| ACETYL METHYL CARBINOL | 5,000 |
| LINALOOL | 20,000 |
| PHENIRAT^{®} | 4,000 |
| CLOVE LEAF OIL DECOL. | 40,000 |
| EUGENOL NAT. | 250,000 |
| ANISIC ALDEHYDE PURE | 5,000 |
| ETHYL VANILLIN | 30,000 |
| VANILLIN | 5,000 |
| METHYL CINNAMIC ALDEHYDE ALPHA | 50,000 |
| CINNAMIC ALDEHYDE | 160,000 |
| BENZALDEHYDE DD | 5,000 |
| COUMARIN | 30,000 |
| AGRUMEX LC | 30,000 |
| BENZYL BENZOATE M | 208,000 |
| Ethyl 2-methylbenzoate | 20,000 |
| Total | 1.000,000 |

According to an expert panel, the addition of ethyl 2-methylbenzoate enhances the warm spices of the fragrance composition. Especially the Nutmeg and Clove character is enhanced by this effect. The Pumpkin part of the fragrance harmonized wonderfully with the mix of the boosted spices. The perfume oil was evaluated in a candle in an amount of 3%

| | |
|---|---|
| PI29848 PINANOL | 10,000 |
| ALDEHYDE C8 | 15,000 |
| LINOLAL | 8,000 |
| DIHYDRO MYRCENOL | 35,000 |
| TERPINYL ACETATE | 188,000 |
| LEMON OIL TERPENES | 30,000 |
| AGRUNITRILE | 40,000 |
| CITRONITRILE | 80,000 |
| CITRYLAL | 10,000 |
| EUCALYPTUS OIL GLOBULUS 80/85% | 50,000 |
| ISOBUTYLMENTHONE | 2,000 |
| BORNYL ACETATE L CRYST. | 3,000 |
| ISOBORNYL ACETATE | 25,000 |
| FENCHOL | 5,000 |
| ISOBORNEOL | 15,000 |
| LINALOOL | 50,000 |
| CITRONELLOL 950 | 200,000 |
| GERANIOL PRIME (drum) | 85,000 |
| NEROLEX (drum) | 20,000 |
| GERANYL ACETATE PURE | 15,000 |
| EUGENOL NAT. | 10,000 |
| ISOEUGENOL | 2,000 |
| HERBAFLORAT | 30,000 |
| PATCHOULI OIL DECOL. | 4,000 |
| FLOWERPOOL 0,1% DPG | 8,000 |
| ELEMI EXTRACT STANDARD | 40,000 |
| Ethyl 2-methylbenzoate | 20,000 |
| Total | 1.000,000 |

According to an expert panel, the addition of ethyl 2-methylbenzoate is covering the Citronella character of this notes and makes it more spicy sharp Ginger like. The Clove note is enhanced by ethyl 2-methylbenzoate and brings the warm undertone to the Ginger note. The perfume oil was evaluated in soap in an amount of 1,2%.

| | |
|---|---|
| ALDEHYDE C11 UNDECANAL 10% DPG | 2,000 |
| ALDEHYDE C12 LAURIC | 1,000 |
| HEXENYL ACETATE CIS-3 10% DPG | 6,000 |
| VERTOCITRAL 10% DPG | 2,000 |
| PHENYLACETALDEHYDE 10% DPG | 5,000 |
| DIHYDRO MYRCENOL | 50,000 |
| ORANGE OIL TERPENES | 3,000 |
| PINENE ALPHA LAEVO NAT. | 3,000 |
| TERPINOLENE DEXTRO | 1,000 |
| ROSEMARY OIL RCO SPAN. | 7,500 |
| ROSEMARY OIL TUN. | 5,000 |
| EUCALYPTUS OIL GLOBULUS 80/85% | 115,000 |
| MENTHA ARV. OIL RECT. IND. | 10,000 |
| MENTHOL RAC. | 2,000 |
| MENTHYL ACETATE RAC. | 1,000 |
| CYMOL PARA SUPRA | 2,000 |
| TURPENTINE OIL PORTUG. | 3,000 |
| BORNYL ACETATE L CRIST. | 2,000 |
| BORNEOL L/ ISOBORNEOL 65/35 | 1,000 |
| CAMPHENE | 2,500 |
| CAMPHOR DL | 7,000 |
| CANTHOXAL | 5,000 |
| FLOROSA BM / PYRANOL | 15,000 |
| MAJANTOL^{®} | 10,000 |
| LINALOOL | 35,000 |
| TERPINEOL, ALPHA-, SUPRA (drum) | 1,000 |
| PHENYLETHYL ALCOHOL | 50,000 |
| CITRONELLOL 950 | 40,000 |
| GERANIOL PRIME (drum) | 15,000 |
| DAMASCENONE 10% DPG | 3,000 |
| HEXYL CINNAMIC ALDEHYDE ALPHA | 150,000 |
| BENZYL SALICYLATE | 10,000 |
| HEXYL SALICYLATE | 20,000 |
| IONONE ALPHA | 10,000 |
| ISORALDEINE 70 | 20,000 |
| PHENYL PROPYL ALCOHOL | 10,000 |
| ORYCLON SPECIAL | 20,000 |
| ETHYLENE BRASSYLATE | 20,000 |
| GLOBALIDE^{®} | 30,000 |
| DIPROPYLENE GLYCOL | 280,000 |
| Ethyl 2-methylbenzoate | 5,000 |
| Total | 1.000,000 |

According to an expert panel, the addition of ethyl 2-methylbenzoate is giving a boosting function to the aromatic rosemary body and lifts up this effect to the Eucalyptus top note. It makes the fragrance stronger, livelier and it is balancing the fragrance profiles. The perfume oil was evaluated in liquid soap in an amount of 0,5%.

| | |
|---|---|
| STYRALYL ACETATE | 5,000 |
| BERGAMOT OIL BERGAPTEN FREE | 70,000 |
| MANDARIN OIL EXPRESSED MADA | 7,000 |
| AMAROCIT^{®} | 25,000 |
| METHYL ANTHRANILATE 10% DPG | 3,000 |
| ROSEMARY OIL NORTH AFRICA | 40,000 |
| CARDAMOM OIL | 7,000 |
| PINK PEPPER LEAF OIL MADA | 20,000 |
| SAFRALEINE | 20,000 |
| ETHYL LINALOOL | 40,000 |
| PHENYLETHYL ACETATE | 5,000 |
| PHENYLETHYL ALCOHOL | 30,000 |
| DAMASCENONE 10% DPG | 4,000 |
| HEDIONE | 80,000 |
| HEXENYL SALICYLATE CIS-3 | 10,000 |
| ETHYL VANILLIN | 2,500 |
| VANILLIN | 7,000 |
| COUMARIN | 25,000 |
| CEDARWOO OIL SUPER RECT. | 20,000 |
| ISO E SUPER NON DISCOLORING | 300,000 |
| VETIVERYL ACETATE EXTRA | 25,000 |
| FLOWERPOOL 0,1% DPG | 4,000 |
| ELEMI OIL | 13,000 |
| OLIBANUM OIL | 8,000 |
| AMBROXIDE | 6,500 |
| GLOBALIDE^{®} | 130,000 |
| MUSCENONE | 27,000 |
| MACROLIDE^{®} SUPRA | 27,000 |
| DIPROPYLENE GLYCOL | 10,000 |
| Ethyl 2-methylbenzoate | 4,000 |
| Total | 1.000,000 |

According to an expert panel, the addition of ethyl 2-methylbenzoate enhanced the Fougère note in this Fine Fragrance by its boosting function. Furthermore, it makes the fragrance more aromatic. It has a spicy peppery body which is livelier in combination with ethyl 2-methylbenzoate. The perfume oil was evaluated in alcoholin an amount of 010%.

## Claims

1. Use of ethyl 2-methylbenzoate as a fragrance.

2. Use of ethyl 2-methylbenzoate in fragrance compositions to obtain at least one of the following fragrance notes: strong, clean, floral, natural, volume, fruity, fresh, spicy, durable, complex, fine, green, less sweaty, less animalic, less mushroomy and/or less terpenic.

3. Use according to claim 1 and/or 2 for imparting, modifying and/or enhancing an aromatic and/or spicy odor note.

4. Use according to claim 1 and/or 2 for enhancing an aromatic and/or spicy odor note.

5. Fragrance composition comprising or consisting of ethyl 2-methylbenzoate and at least one further fragrance.

6. Fragrance composition according to claim 5, wherein the weight ratio of ethyl 2-methylbenzoate to the total amount of other fragrances is in the range of 1:10 to 1:100.

7. Fragrance composition according to claim 5 and/or 6, wherein the amount of ethyl 2-methylbenzoate is in the range of 0.01 to 50% by weight, based on the total weight of the fragrance composition.

8. Fragrance composition according to claim 5 and/or 6, wherein the amount of ethyl 2-methylbenzoate is in the range of 0.01 to 1.5% by weight, based on the total weight of the fragrance composition.

9. Fragrance composition according to at least one of claims 5 to 8, wherein the at least one further fragrance is selected from the group consisting of (1R,2S,4R)-1,7,7-Trimethyl-bicyclo[2.2.1]heptan-2-ol, (1S,2R,4S)-1,7,7-Trimethyl-bicyclo[2.2.1]heptan-2-ol, (1S,2S,4S)-1,7,7-Trimethyl-bicyclo[2.2.1]heptan-2-ol, (1R,2R4R)-1,7,7-Trimethyl-bicyclo[2.2.1]heptan-2-ol, [2-(2-methylbutan-2-yl)cyclohexyl] acetate, Terpinen-4-ol, Isobornyl Acetate, 6,6-Dimethyl-2-methyli-denebicyclo[3.1.1]heptanePin-2(10)-ene, (1S,5S)-2,6,6-Trimethylbicyclo[3.1.1]hept-2-ene ((-)-α-Pinene), 1,7,7-Trimethylbicyclo[2.2.1]heptan-2-one, Anethol, Cinnamic Aldehyde, Methyl-4(or 1)-isopropyl-1(or 4)-methylbicyclo[2.2.2]oct-5-ene-2-carboxylate, (1R,4E,9S)-4,11,11-Trimethyl-8-methyli-denebicyclo[7.2.0]undec-4-ene, Isoeugenyl Methyl Ether, 4-Allyl-2-methoxyphenol, 2-butyl-4,4,6-trimethyl-1,3-dioxane, Cuminic Aldehyde, 2,3,3-Trimethylin-dan-1-one, Pinanol, Dihydro Myrcenol, Terpinyl acetate, 3-methyl-5-phenylpent-2-enenitrile, Geranyl acetate, benzaldehyde, ionone alpha, benzyl salicylate, phenylethyl alcohol, 3,7-Dimethyloct-6-en-1-ol, Methyldihydrojasmonat, (5E)-3-methylcyclopentadec-5-en-1-one, (3aR,5aS,9aS,9bR)-3a,6,6,9a-Tetramethyl-dodecahydronaphtho[2,1-b]furan, 3-Ethoxy-4-hydroxybenzaldehyd, 4-Hydroxy-3-methoxybenzaldehyd.

10. Use of a fragrance composition according to any one of claims 5 to 9 to modify an existing fragrance within the fragrance composition to make it stronger, cleaner, more floral, more natural, more volume, fruitier, fresher, more durable, more complex, finer, greener, less sweaty, less animalic, less mushroomy and/or less terpenic and/or to enhance an aromatic and/or spicy fragrance note of the existing fragrance.

11. Method for modifying an existing fragrance to make it stronger, cleaner, more floral, more natural, more volume, fruitier, fresher, more durable, more complex, finer, greener, less sweaty, less animalic, less mushroomy and/or less terpenic and/or to enhance an aromatic and/or spicy fragrance note, comprising or consisting of the following steps:
(a) providing ethyl 2-methylbenzoate,
(b) providing at least one further fragrance,
(c) adding the ethyl 2-methylbenzoate to the at least one further fragrance in a sensory effective amount sufficient to modify the at least one further fragrance to make it stronger, cleaner, more floral, more natural, more volume, fruitier, fresher, more durable, more complex, finer, greener, less sweaty, less animalic, less mushroomy and/or less terpenic and/or to enhance an aromatic and/or spicy fragrance note.

12. Perfumed product comprising ethyl 2-methylbenzoate and two or more other fragrances and optionally other ingredients, **characterized in that** the blend comprises ethyl 2-methylbenzoate in a quantity which
(a) modifies the olfactory properties of the two or more other fragrances to make it stronger, cleaner, more floral, more natural, more volume, fruitier, fresher, more durable, more complex, finer, greener, less sweaty, less animalic, less mushroomy and/or less terpenic and/or
(b) enhances the aromatic and/or spicy note of the olfactory properties of the two or more other fragrances.

13. The perfumed product according to claim 12, wherein the product is selected from cosmetic, hygiene and/or household articles.

14. The perfumed product according to claim 12, wherein the product is selected from an alcoholic perfume, a personal hygiene product or a cleaning or care product for use in the home.

## Patentansprüche

1. Verwendung von Ethyl 2-methylbenzoat als Duftstoff.

2. Verwendung von Ethyl 2-methylbenzoat in Duftstoffzusammensetzungen um mindestens eine der folgenden Duftnoten zu erzielen: stark, rein, blumig, natürlich, voluminös, fruchtig, frisch, würzig, langanhaltend, komplex, fein, grün, weniger schweißig, weniger animalisch, weniger pilzartig und/oder weniger terpenartig.

3. Verwendung nach Anspruch 1 und/oder 2 zum Verleihen, Modifizieren und/oder Verstärken einer aromatischen und/oder würzigen Duftnote.

4. Verwendung nach Anspruch 1 und/oder 2 zur Verstärkung einer aromatischen und/oder würzigen Duftnote.

5. Duftstoffzusammensetzung umfassend oder bestehend aus Ethyl 2-methylbenzoat und mindestens einem weiteren Duftstoff.

6. Duftstoffzusammensetzung nach Anspruch 5, wobei das Gewichtsverhältnis von Ethyl-2-methylbenzoat zur Gesamtmenge der übrigen Duftstoffe im Bereich von 1:10 bis 1:100 liegt.

7. Duftstoffzusammensetzung nach Anspruch 5 und/oder 6, wobei die Menge an Ethyl-2-methylbenzoat, bezogen auf das Gesamtgewicht der Duftstoffzusammensetzung, im Bereich von 0,01 bis 50 Gew.-% liegt.

8. Duftstoffzusammensetzung nach Anspruch 5 und/oder 6, wobei die Menge an Ethyl-2-methylbenzoat, bezogen auf das Gesamtgewicht der Duftstoffzusammensetzung, im Bereich von 0,01 bis 1,5 Gew.-% liegt.

9. Duftstoffzusammensetzung nach mindestens einem der Ansprüche 5 bis 8, wobei der mindestens eine weitere Duftstoff ausgewählt ist aus der Gruppe bestehend aus (1R,2S,4R)-1,7,7-Trimethyl-bicyclo[2.2.1]heptan-2-ol, (1S,2R,4S)-1,7,7-Trimethyl-bicyclo[2.2.1]heptan-2-ol, (1S,2S,4S)-1,7,7-Trimethyl-bicyclo[2.2.1]heptan-2-ol, (1R,2R,4R)-1,7,7-Trimethyl-bicyclo[2.2.1]heptan-2-ol, [2-(2-Methylbutan-2-yl)cyclohexyl]acetat, Terpinen-4-ol, Isobornylacetat, 6,6-Dimethyl-2-methylidenbicyclo[3.1.1]heptanPin-2(10)-en, (1S,5S)-2,6,6-Trimethylbicyclo[3.1.1]hept-2-en ((-)-α-Pinen), 1,7,7-Trimethylbicyclo[2.2.1]heptan-2-on, Anethol, Zimtaldehyd, Methyl-4(oder 1)-isopropyl-1(oder 4)-methylbicyclo[2.2.2]oct-5-en-2-carboxylat, (1R,4E,9S)-4,11,11-Trimethyl-8-methylidenbicyclo[7.2.0]undec-4-en, Isoeugenylmethylether, 4-Allyl-2-methoxyphenol 2-Butyl-4,4,6-trimethyl-1,3-dioxan, Cuminaldehyd, 2,3,3-Trimethylindan-1-on, Pinanol, Dihydromyrcenol, Terpinylacetat, 3-Methyl-5-phenylpent-2-ennitril, Geranylacetat, Benzaldehyd, Ionon-alpha, Benzylsalicylat, Phenylethylalkohol, 3,7-Dimethyloct-6-en-1-ol, Methyldihydrojasmonat, (5E)-3-Methylcyclopentadec-5-en-1-on, (3aR,5aS,9aS,9bR)-3a,6,6,9a-Tetramethyldodecahydronaphtho[2,1-b]furan, 3-Ethoxy-4-hydroxybenzaldehyd, 4-Hydroxy-3-methoxybenzaldehyd.

10. Verwendung einer Duftstoffzusammensetzung nach einem der Ansprüche 5 bis 9 zur Modifizierung eines bestehenden Duftes innerhalb der Dufstoffzusammensetzung, um ihn stärker, reiner, blumiger, natürlicher, voluminöser, fruchtiger, frischer, haltbarer, komplexer, feiner, grüner, weniger schweißig, weniger animalisch, weniger pilzartig und/oder weniger terpenhaltig zu machen und/oder um eine aromatische und/oder würzige Duftnote des bestehenden Duftes zu verstärken.

11. Verfahren zur Modifizierung eines bestehenden Duftstoffs, um ihn intensiver, reiner, blumiger, natürlicher, voluminöser, fruchtiger, frischer, haltbarer, komplexer, feiner, grüner, weniger schweißig, weniger animalisch, weniger pilzartig und/oder weniger terpenartig zu machen und/oder um eine aromatische und/oder würzige Duftnote zu verstärken, umfassend oder bestehend aus den folgenden Schritten:
(a) Bereitstellen von Ethyl-2-methylbenzoat,
(b) Bereitstellen mindestens eines weiteren Duftstoffs,
(c) Hinzufügen des Ethyl-2-methylbenzoats zu dem mindestens einen weiteren Duftstoff in einer sensorisch wirksamen Menge, die ausreicht, um den mindestens einen weiteren Duftstoff so zu modifizieren, dass er intensiver, reiner, blumiger, natürlicher, voluminöser, fruchtiger, frischer, haltbarer, komplexer, feiner, grüner, weniger schweißig, weniger animalisch, weniger pilzartig und/oder weniger terpenartig wird und/oder um eine aromatische und/oder würzige Duftnote zu verstärken.

12. Parfümiertes Produkt, umfassend Ethyl-2-methylbenzoat und zwei oder mehr andere Duftstoffe und gegebenenfalls weitere Inhaltsstoffe, **dadurch gekennzeichnet, dass** die Mischung Ethyl-2-methylbenzoat in einer Menge enthält, die
(a) die olfaktorischen Eigenschaften der zwei oder mehr anderen Duftstoffe so verändert, dass sie stärker, reiner, blumiger, natürlicher, voluminöser, fruchtiger, frischer, länger anhaltend, komplexer, feiner, grüner, weniger schweißig, weniger animalisch, weniger pilzartig und/oder weniger terpenhaltig werden und/oder
(b) die aromatische und/oder würzige Note der olfaktorischen Eigenschaften der zwei oder mehr anderen Duftstoffe verstärkt.

13. Das parfümierte Produkt nach Anspruch 12, wobei das Produkt aus Kosmetik-, Hygiene- und/oder Haushaltsartikeln ausgewählt ist.

14. Das parfümierte Produkt nach Anspruch 12, wobei das Produkt aus einem alkoholischen Parfüm, einem Körperpflegeprodukt oder einem Reinigungs- oder Pflegeprodukt für den Haushalt ausgewählt ist.

## Revendications

1. Utilisation du 2-méthylbenzoate d'éthyle comme parfum.

2. Utilisation du 2-méthylbenzoate d'éthyle dans des compositions parfumées pour obtenir au moins l'une des notes parfumées suivantes : forte, propre, florale, naturelle, volumineuse, fruitée, fraîche, épicée, durable, complexe, fine, verte, moins suintante, moins animale, moins champignon et/ou moins terpénique.

3. Utilisation selon la revendication 1 et/ou 2 pour conférer, modifier et/ou renforcer une note aromatique et/ou épicée.

4. Utilisation selon la revendication 1 et/ou 2 pour renforcer une note aromatique et/ou épicée.

5. Composition parfumée comprenant ou consistant en du 2-méthylbenzoate d'éthyle et au moins un autre parfum.

6. Composition parfumée selon la revendication 5, dans laquelle le rapport pondéral du 2-méthylbenzoate d'éthyle à la quantité totale des autres parfums est compris entre 1:10 et 1:100.

7. Composition parfumée selon la revendication 5 et/ou 6, dans laquelle la quantité de 2-méthylbenzoate d'éthyle est comprise entre 0,01 et 50 % en poids, par rapport au poids total de la composition parfumée.

8. Composition parfumée selon la revendication 5 et/ou 6, dans laquelle la quantité de 2-méthylbenzoate d'éthyle est comprise entre 0,01 et 1,5 % en poids, par rapport au poids total de la composition parfumée.

9. Composition parfumée selon au moins l'une des revendications 5 à 8, dans laquelle le au moins un autre parfum est choisi dans le groupe constitué par le (1R,2S,4R)-1,7,7-triméthyl-bicyclo[2.2.1]heptan-2-ol, le (1S,2R,4S)-1,7,7 -triméthyl-bicyclo[2.2.1]heptan-2-ol, (1S,2S,4S)-1,7,7-triméthyl-bicyclo[2.2.1]heptan-2-ol, (1R,2R,4R)-1,7,7 -triméthyl-bicyclo[2.2.1]heptan-2-ol, acétate de [2-(2-méthylbutan-2-yl)cyclohexyle], terpinène-4-ol, acétate d'isobornyle, 6,6-diméthyl-2-méthylidènebicyclo[3.1.1]heptanePin-2(10)-ène, (1S,5S)-2,6,6-triméthylbicyclo[3.1.1]hept-2-ène ((-)-α-pinène), 1,7,7-triméthylbicyclo[2.2.1]heptan-2-one, anéthol, aldéhyde cinnamique, méthyl-4(ou 1)-isopropyl-1(ou 4)-méthylbicyclo[2.2.2]oct-5-ène-2-carboxylate, (1R,4E,9S)-4,11,11-triméthyl-8-méthylidènebicyclo[7.2.0]undéc-4-ène, éther méthylique d'isoeugénol, 4-allyl-2-méthoxyphénol, 2-butyl-4,4,6-triméthyl-1,3-dioxane, aldéhyde cuminique, 2,3,3-triméthylindan-1-one, pinanol, dihydro myrcénol, acétate de terpinyle, 3-méthyl-5-phénylpent-2-énonitrile, acétate de géranyle, benzaldéhyde, ionone alpha, salicylate de benzyle, alcool phényléthylique, 3,7-diméthyloct-6-én-1-ol, méthyldihydrojasmonate, (5E)-3-méthylcyclopentadéc-5-én-1-one, (3aR,5aS,9aS,9bR)-3a, 6,6,9a-tétraméthyldodécahydronaphto[2,1-b]furane, 3-éthoxy-4-hydroxybenzaldéhyde, 4-hydroxy-3-méthoxybenzaldéhyde.

10. Utilisation d'une composition parfumée selon l'une quelconque des revendications 5 à 9 pour modifier un parfum existant dans la composition parfumée afin de le rendre plus fort, plus pur, plus floral, plus naturel, plus volumineux, plus fruité, plus frais, plus durable, plus complexe, plus fin, plus vert, moins transpirant, moins animal, moins champignon et/ou moins terpénique et/ou pour rehausser une note aromatique et/ou épicée du parfum existant.

11. Procédé pour modifier un parfum existant afin de le rendre plus fort, plus pur, plus floral, plus naturel, plus volumineux, plus fruité, plus frais, plus durable, plus complexe, plus fin, plus vert, moins suintant, moins animal, moins champignon et/ou moins terpénique et/ou pour rehausser une note aromatique et/ou épicée, comprenant ou consistant en les étapes suivantes :
(a) fournir du 2-méthylbenzoate d'éthyle,
(b) fournir au moins un autre parfum,
(c) ajouter le 2-méthylbenzoate d'éthyle à au moins un autre parfum en une quantité efficace sur le plan sensoriel suffisante pour modifier au moins un autre parfum afin de le rendre plus fort, plus pur, plus floral, plus naturel, plus volumineux, plus fruité, plus frais, plus durable, plus complexe, plus fin, plus verte, moins transpirante, moins animale, moins champignon et/ou moins terpénique et/ou pour rehausser une note aromatique et/ou épicée.

12. Produit parfumé comprenant du 2-méthylbenzoate d'éthyle et deux ou plusieurs autres parfums et éventuellement d'autres ingrédients, **caractérisé en ce que** le mélange comprend du 2-méthylbenzoate d'éthyle en une quantité qui
(a) modifie les propriétés olfactives des deux ou plusieurs autres parfums pour les rendre plus forts, plus propres, plus floraux, plus naturels, plus volumineux, plus fruités, plus frais, plus durables, plus complexes, plus fins, plus verts, moins suintants, moins animaux, moins champignons et/ou moins terpéniques et/ou
(b) renforce la note aromatique et/ou épicée des propriétés olfactives des deux ou plusieurs autres parfums.

13. Produit parfumé selon la revendication 12, dans lequel le produit est choisi parmi les articles cosmétiques, d'hygiène et/ou ménagers.

14. Produit parfumé selon la revendication 12, dans lequel le produit est choisi parmi un parfum alcoolisé, un produit d'hygiène personnelle ou un produit de nettoyage ou d'entretien à usage domestique.
